(19) Europäisches Patentamt
European Patent Office
Office européen des brevets

(11) Numéro de publication: **0 320 346**
**A2**

(12) **DEMANDE DE BREVET EUROPEEN**

(21) Numéro de dépôt: **88403044.6**

(51) Int. Cl.[4]: **C 07 C 37/055**

(22) Date de dépôt: **02.12.88**

(30) Priorité: **11.12.87 FR 8717323**

(43) Date de publication de la demande:
**14.06.89 Bulletin 89/24**

(84) Etats contractants désignés:
**AT BE CH DE ES FR GB GR IT LI LU NL SE**

(71) Demandeur: **RHONE-POULENC CHIMIE**
**25, quai Paul Doumer**
**F-92408 Courbevoie Cédex (FR)**

(72) Inventeur: **Gubelmann, Michel**
**39, boulevard des Belges**
**F-69006 Lyon (FR)**

(74) Mandataire: **Le Pennec, Magali et al**
**RHONE-POULENC INTERSERVICES Service Brevets**
**Chimie 25, Quai Paul Doumer**
**F-92408 Courbevoie Cédex (FR)**

(54) **Procédé de préparation de dérivés aromatiques hydroxylés par la réaction de Bayer-Villiger.**

(57) La présente invention concerne un procédé de préparation de dérivés aromatiques hydroxylés par la réaction de Baeyer-Villiger caractérisé en ce que
dans une première étape on forme une solution d'acide percarboxylique,
dans une deuxième étape on ajoute à la solution précédente une arylcétone ou un aldéhyde aromatique.

Les dérivés hydroxylés obtenus sont utilisés dans toute l'industrie chimique des polymères, pharmaceutique ou phytosanitaire.

EP 0 320 346 A2

## Description

## PROCEDE DE PREPARATION DE DERIVES AROMATIQUES HYDROXYLES PAR LA REACTION DE BAEYER-VILLIGER

La présente invention concerne un procédé de préparation de dérivés aromatiques hydroxylés. Elle concerne plus particulièrement un procédé de préparation de dérivés aromatiques hydroxylés à partir de cétones aromatiques ou d'aldéhydes aromatiques par la réaction de Baeyer-Villiger.

Il est connu depuis déjà très longtemps tel que décrit par exemple par VON E. DOERING et al. dans le Journal of the American Chemical Society 72, 5515-5518 de préparer des phénols à partir de cétones aromatiques par la technique de Baeyer-Villiger en utilisant comme peracide l'acide peracétique, la réaction étant catalysée par l'addition d'acide sulfurique.

De nombreuses publications depuis le début du siècle ont décrit la réaction de Baeyer-Villiger sur diverses cétones telles que les acétophénones, les cétones cycliques, dans des conditions variées tant en ce qui concerne le catalyseur acide (acides sulfurique, phosphorique) que l'absence d'eau et de péroxyde d'hydrogène dans la solution de peracétique.

Il est encore connu d'après la demande de brevet européen publiée sous le numéro EP 178929 de préparer l'hydroquinone ou ses dérivés à partir de parahydroxyacétophénone par la réaction de Baeyer-Villiger à l'aide d'acide peracétique comme agent oxydant et en présence d'acide sulfurique en quantité inférieure à 0,1 % en poids par rapport à l'acide peracétique employé. La solution d'acide peracétique est toujours ajoutée à la cétone (description page 4 dernière ligne et page 9 ligne 29 et tous les exemples).

La température réactionnelle est comprise dans la description entre 20 et 150°C, mais dans tous les exemples elle est de 60°C.

Les conditions réactionnelles c'est-à-dire l'introduction de l'acide peracétique pur et distillé dans la solution contenant le réactif cétonique, ainsi que la purification nécessaire de l'acide peracétique par distillation ont toujours fait reculer l'industrie quant à l'utilisation de cette technique. Les normes de sécurité exigées par la distillation de réactifs explosifs comme les peracides nous ont amené à écarter la méthode décrite dans cette demande de brevet.

D'autre part, au-delà de 80°C, la détonabilité de l'acide peracétique est encore aggravée.

Il est aussi connu de purifier les peracides tel que décrit dans le brevet français FR 2 359 132 par une suite d'extractions à contre courant d'abord par le benzène puis par l'eau suivi ensuite par l'ajout d'un stabilisant. La solution de peracide obtenue contient en fin d'extraction une quantité d'eau et de peroxyde d'hydrogène non négligeable. Le procédé est inutilisable dans le cadre de la présente invention car la pureté du peracide n'est pas suffisante et le procédé est beaucoup trop compliqué. En fin de réaction il subsiste dans la solution d'acide percarboxylique du peroxyde d'hydrogène et de l'eau.

L'industrie est donc depuis longtemps à la recherche d'une méthode permettant d'une part d'utiliser la réaction de Baeyer-Villiger sans distiller l'acide peracétique, sans utiliser une technique d'extraction liquide-liquide, d'autre part permettant de travailler à une température inférieure à 80°C.

La demande de brevet précédemment citée c'est-à-dire la demande européenne publiée sous le numéro EP 178929 est en outre strictement limitée à la préparation des hydroquinones. Or l'industrie cherche depuis longtemps à préparer par la technique de Baeyer-Villiger des dérivés phénoliques plus difficiles à préparer que sont les parahalogénophénols et particulièrement le parafluorophénol qui n'a jamais été préparé par cette technique.

La présente invention concerne donc un procédé de préparation de dérivés aromatiques hydroxylés par la réaction de Baeyer-Villiger caractérisé en ce que

dans une première étape on prépare une solution d'acide percarboxylique par addition d'anhydride carboxylique à une solution de peroxyde d'hydrogène dans un solvant halogéné en présence d'un acide fort, dans une deuxième étape on ajoute une arylcétone et/ou un aldéhyde aromatique à la solution précédente et on maintient le milieu à une température inférieure ou égale à 40°C.

L'acide fort peut être inorganique ou organique. Il pourra se présenter sous forme homogène ou hétérogène. On peut citer notamment l'acide sulfurique, l'acide méthanesulfonique, trifluorométhanesulfonique, l'acide trifluoroacétique, l'acide phosphorique, des résines cationiques de type Amberlyst® et Nafion®

.

L'acide percarboxylique est de préférence un acide non halogéné ayant un à trois atomes de carbone et tout particulièrement l'acide performique ou l'acide peracétique.

La réaction de Baeyer-Villiger peut être schématisée selon l'équation réactionnelle suivante, lorque l'on utilise l'acide peracétique :

$$R'-Ar-\underset{\underset{O}{\|}}{C}-R + CH_3CO_2OH \rightarrow R'-ArO\underset{\underset{O}{\|}}{C}-R + CH_3CO_2H$$

$$R'-ArO\underset{\underset{O}{\|}}{C}-R + H_2O \rightarrow R'-Ar-OH + RCOOH$$

Selon le brevet EP178929 la préparation de l'acide peracétique pur, anhydre et exempt de peroxyde d'hydrogène à partir de l'acide peracétique commercial contenant 40 % en poids d'acide peracétique, 13 % d'eau et environ 5 % de péroxyde d'hydrogène et 1 % d'acide sulfurique est réalisée par distillation à 40°C, sous pression réduite pour éviter au maximum les risques d'explosion.

Selon le brevet FR2359132 la préparation des péracides purs est réalisée par extraction à contre courant du péracide commercial avec du benzène.

Ces techniques présentent toujours des risques. Aussi la présente invention a permis d'éviter ces dangers en préparant directement dans l'enceinte réactionnelle l'acide percarboxylique.

Selon la présente invention on introduit dans le réacteur le catalyseur acide minéral ou organique dans un solvant aliphatique halogéné ayant 1 à 6 atomes de carbone et de préférence chloré. On préfère tout particulièrement utiliser les solvants aliphatiques ayant 1 à 2 atomes de carbone et tout spécialement le dichloroéthane.

On ajoute ensuite le péroxyde d'hydrogène puis l'anhydride carboxylique. L'addition est réalisée de façon à ce que la quantité totale d'anhydride carboxylique soit en excès par rapport au péroxyde d'hydrogène de façon à ne pas laisser subsister dans la solution finale ni eau ni péroxyde d'hydrogène. On prépare ainsi des solutions d'acide percarboxylique dans le dichloroéthane ne dépassant pas 35 % en poids et de préférence comprise entre 15 et 25 % et tout particulièrement des solutions à 20 % en poids.

A cette solution sans aucun traitement accessoire est ajoutée l'arylcétone ou l'aldéhyde aromatique.

L'arylcétone utilisée répond à la formule générale (I)

$(R')_n$-Ar-CO-R (I)

dans laquelle :

Ar représente un radical mono ou polycyclique ou hétérocyclique,

R′ représente un motif choisi parmi les radicaux alkyle, contenant 1 à 4 atomes de carbone, alcoxy contenant 1 à 4 atomes de carbone, hydroxy, halogéno, perhalogénoalkyle, perhalogénalcoxy, acyloxy, alcoxycarbonyl, nitro, phényl et phénoxy, éventuellement substitués par un groupe hydroxyle, halogéno, formyl, acyloxy.

n est un nombre entier égal à 0, 1 ou 2,

R représente l'hydrogène, un groupe alkyle contenant 1 à 4 atomes de carbone, un groupe phényl.

On préfère utiliser les arylcétones dans lesquelles Ar représente un radical benzénique, R représente l'hydrogène ou un groupe méthyle et R′ le fluor.

Parmi les composés de formule (I) on peut citer notamment :

- composés dans lesquels R = H
. le benzaldéhyde,
. les ortho, méta et para-méthylbenzaldéhydes (tolualdéhydes),
. les ortho, méta et para-fluorobenzaldéhydes,
. les ortho, méta et para-chlorobenzaldéhydes,
. les ortho, méta et para-nitrobenzaldéhydes,
. les ortho, méta et para-trifluorométhylbenzaldéhydes,
. les ortho, méta et para-phénylbenzaldéhydes,
. les ortho, méta et para-(hydroxy-4-phényl)benzaldéhydes,
. les biphényl-4-4′-dicarboxaldéhyde,
. l'acétyl-4-biphényl-4′-carboxaldéhyde,
. les ortho, méta et para-hydroxybenzaldéhydes,
. les ortho, méta et para-méthoxybenzaldéhydes,
. les ortho, méta et para-acétoxybenzaldéhydes,
. les ortho, méta et para-phénoxybenzaldéhydes,
. les ortho, méta et para-(hydroxy-4-phénoxy)benzaldéhydes,
. les ortho, méta et para-diphényléther-4-4′-dicarboxaldéhydes,
. l'acétyl-4-diphényléther-4′-carboxaldéhyde,
. le naphth-2-aldéhyde,
. le méthoxy-4-méthyl-3-benzaldéhyde,
. le méthoxy-4-méthyl-2-benzaldéhyde,
. l'indole-3-(carboxaldéhyde-6)-carboxylate de méthyle,
. l'indole-3-(carboxaldéhyde-5)-carboxylate de méthyle,
. - composés dans lesquels R = $CH_3$
. l'acétophénone,
. les ortho, méta et para-méthylacétophénones,
. les ortho, méta et para-fluoroacétophénones,
. les ortho, méta et para-chloroacétophénones,
. les ortho, méta et para-nitroacétophénones,
. les ortho, méta et para-trifluorométhylacétophénones,
. les ortho, méta et para-phénylacétophénones,
. les ortho, méta et para-(hydroxy-4-phényl) acétophénones,
. l'acétyl-4-biphényl-4′-carboxaldéhyde,
. le diacétyl-4,4′-biphényle,
. les ortho, méta et para-hydroxyacétophénones,
. les ortho, méta et para-méthoxyacétophénones,
. les ortho, méta et para-acétoxyacétophénones,
. les ortho, méta et para-phénoxyacétophénones,
. les ortho, méta et para-(hydroxy-4-phénoxy)acétophénones,

. l'acétyl-4-diphényléther-4'-carboxaldéhyde,
. le diacétyl-4,4'-diphényléther,
. l'acétyl-2-naphthalène,
. la méthoxy-4-méthyl-3-acétophénone,
. la méthoxy-4-méthyl-2-acétophénone,
. l'indole-3-(acétyl-6)-carboxylate de méthyle,
. l'indole-3-(acétyl-5)-carboxylate de méthyle,
- composés dans lesquels R représente un groupe phényl
. les ortho, méta et para-méthylbenzophénones,
. les ortho, méta et para-fluorobenzophènones,
. les ortho, méta et para-chlorobenzophénones,
. les ortho, méta et para-nitrobenzophénones,
. les ortho, méta et para-trifluorométhylbenzophénones,
. les ortho, méta et para-phénylbenzophénones,
. les ortho, méta et para-(hydroxy-4-phényl)benzophénones,
. le benzoyl-4-biphényl-4'-carboxaldéhyde,
. l'acétyl-4-benzoyl-4'-biphényle,
. les ortho, méta et para-hydroxybenzophénones,
. les ortho, méta et para-méthoxybenzophénones,
. les ortho, méta et para-acétoxybenzophénones,
. les ortho, méta et para-phénoxybenzophénones,
. les ortho, méta et para-(hydroxy-4-phénoxy)benzophénones,
. le benzoyl-4-diphényléther-4'-carboxaldéhyde,
. le benzoyl-2-naphthalène,
. la méthoxy-4-méthyl-3-benzophénone,
. la méthoxy-4-méthyl-2-benzophénone,
. l'indole-3-(benzoyl-6)-carboxylate de méthyle,
. l'indole-3-(benzoyl-5)-carboxylate de méthyle.

On préfère pour une meilleure mise en oeuvre de l'invention ajouter l'arylcétone en quantité telle que le rapport du nombre de moles d'acide peracétique par équivalent cétonique ou aldéhydique soit compris entre 1 et 5 et de préférence entre 1,2 et 1,3.

On entend par équivalent cétonique ou aldéhyde chaque fonction cétone ou aldéhyde comprise dans chaque molécule. Ainsi une dicétone représentera deux équivalents et exigera au minimum deux moles d'acide péracetique.

Lors de l'addition de l'arylcétone à la solution d'acide peracétique on ajoute avantageusement un catalyseur acide fort homogène choisi parmi : - l'acide sulfurique
- l'acide phosphorique
- l'acide trifluorométhanesulfonique
- l'acide méthanesulfonique
ou hétérogène choisi parmi : - l'Amberlyst® commercialisé par Rohm & Haas
- le Nafion® commercialisé par Dupont

Le Nafion® commercialisé par Du Pont de Nemours est un polymère perfluoré de formule générale :

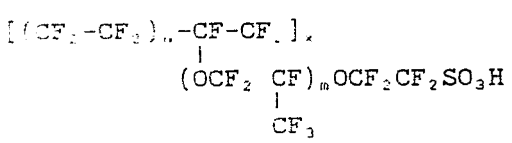

dans laquelle : n est un nombre compris entre 5 et 13,5,
- m est égal à un nombre entier égal à 1,2 ou 3,
- x a une valeur d'environ 1 000

La quantité de catalyseur acide ajoutée est telle que le rapport pondéral du catalyseur à l'acide peracétique soit de préférence compris entre 5 et 20 %.

L'invention sera plus complètement décrite à l'aide des exemples suivants qui ne doivent en aucun cas être considérés comme limitatifs de l'invention.

Dans les exemples les abréviations suivantes signifient :
[O-O]: concentration en peroxydes organiques,
pp : poids/poids,
TT : taux de transformation du produit de départ

$$( = \text{conversion} = \left[\frac{\text{nombre mole transformé}}{\text{nombre mole intial}}\right] \times 100)$$

RT : rendement calculé sur le produit transformé

$$( = \text{sélectivité} = \left[\frac{\text{nombre mole de produit}}{\text{nombre mole de réactif transformé}}\right] \times 100)$$

EXEMPLE 1

Dans un mini-réacteur en téflon de 30 $cm^3$ muni d'un barreau aimanté et d'une tête en verre monocol avec une entrée latérale, on introduit à 25° C 1,58 g (16 mM ; 25,9 % pp) de dichloro-1,2-éthane et 0,15 g (1,5 mM ; 2,5 % pp) d'acide sulfurique concentré. Après homogénéisation du mélange on ajoute 0,64 g d'une solution aqueuse d'eau oxygénée 70 % ($H_2O_2$ : 0,45 g, 13 mM, 7,4 % pp ; $H_2O$ : 0,19 g, 11 mM, 3,1 % pp) à l'aide d'un pousse-seringue pendant 15 minutes et avec une bonne agitation. En maintenant la température à 25° C à l'aide d'un bain eau-glace, on ajoute 2,45 g (24 mM, 40,2 % pp) l'anhydride acétique en 30 minutes également avec un pousse-seringue. On laisse agiter pendant 45 minutes à 25° C. La répartition des espèces péroxydiques est la suivante (% molaires) : $H_2O_2$ 0 %, $AcO_2H$ 95 %, AcOOAc 5 %. La teneur en $AcO_2H$ est de 20 % en poids.

La solution d'acide péracétique est chauffée à 40° C et on introduit 1,28 g (10,3 mM, 20,9 % pp) de parafluorobenzaldéhyde à l'aide d'un pousse-seringue en 45 minutes. Un suivi cinétique des différentes espèces est réalisé par chromatographie liquide haute performance. A la fin de l'injection du substrat on obtient :

TT(p-FØCHO) = 92 %,
RT(p-FØOH) = E RT(p-FØOCHO) + RT(p-FØOH) = 91 %,
RT(p-F$ØCO_2H$) = 9 %.

Si les mêmes conditions de température et d'agitation sont maintenues pendant 75 minutes supplémentaires, (temps de réaction global = 2 heures) on obtient :

TT(p-FØCHO) = 97 %,
RT(p-FØOH) = 89 %,
RT(p-F$ØCO_2H$) = 9 %.

EXEMPLE 2 (= exemple comparatif n° 1)

On procède comme dans l'exemple 1 mais en inversant l'ordre d'addition substrat-oxydant. Ainsi, la solution d'acide paracétique une fois préparée est introduite dans une seringue en verre de 10 $cm^3$ munie d'une double enveloppe (maintenue à environ 0° C par une circulation en continu à travers un bain eau-glace) est ajoutée progressivement au parafluorobenzaldéhyde préalablement placé dans le mini-réacteur en téflon et chauffée à 40° C. Après 4 heures de réaction, on obtient :

TT(p-FØCHO) = 81 %,
RT (p-FØOH) = 69 %
RT(p-F$ØCO_2H$) = 26 %.

EXEMPLES 3 à 7

On procède comme dans l'exemple 1 mais avec différents catalyseurs acides homogènes (cf. tableau 1 ci-joint).

EXEMPLES 8 à 16

On procède comme dans l'exemple 1 mais avec différents catalyseurs acides hétérogènes (cf. tableau 2 ci-joint).

EXEMPLE 17

On procède come dans l'exemple 1 mais en substituant la parafluorobenzaldéhyde par la parafluoroacétophénone et l'acide sulfurique par l'acide trifluorométhanesulfonique (acide triflique). La température est de 40° C et le temps de réaction de 4 heures.

TT(p-FØCOMe) = 85 %,
RT(p-FØOH) = 92 %,
RT(p-FØ$CO_2$H) = 3 %.

EXEMPLES 18 à 22

On procède comme dans l'exemple 17 mais avec différents catalyseurs acides homogènes(cf. tableau 3 ci-joint).

EXEMPLES 23 à 30

On procède comme dans l'exemple 17 mais avec différents catalyseurs acides hétérogènes (cf. tableau 4 ci-joint).

EXEMPLE 31 oxydation du para-phénylbenzaldéhyde en para-phénylphénol (ou monohydroxy-4-biphényle)

On procède comme dans l'exemple 1. La température est de 40° C et le substrat est ajouté en 20 minutes sous forme de solution à 33,4 % pp dans du dichloro-1,2-éthane. Le temps de réaction est de 30 minutes.
TT(ØØCHO) = 85 %,
RT(p.ØØOH) = 91 %,
RT(pØØ$CO_2$H) = 3 %.

EXEMPLE 32 oxydation de la para-phénylacétophénone en para-phénylphénol

On procède comme dans l'exemple 1. La température est de 40° C et le substrat est ajouté en 25 minutes sous forme de solution à 12,8 % pp dans du dichloro-1,2-éthane. Le temps de réaction est de 2 heures.
TT(p-ØØCOMe) = 88 %,
RT(p-ØØOH) = 89 %,
RT(p-ØØ$CO_2$H) = 4 %.

EXEMPLE 33 oxydation du para-phénoxybenzaldéhyde en para-phénoxyphénol

On procède comme dans l'exemple 1. La température est de 40° C et le substrat est ajouté en 6 minutes. Le temps de réaction est de 8 minutes.
TT(p-ØOØCHO) = 95 %,
RT(p-ØOØOH) = 93 %,
RT(p-ØOØ$CO_2$H) = 2 %.

EXEMPLE 34 oxydation de la para-phénoxyacétophénone en para-phénoxyphénol

On procède comme dans l'exemple 1. La température est de 40° C et le substrat est ajouté en 35 minutes sous forme de solution à 49 % pp dans du dichloro-1,2-éthane. Le temps de réaction est de une heure.
TT(p-ØOØ$COCH_3$) = 92 %,
RT(p-ØOØOH) = 92 %,
RT(p-ØOØ$CO_2$H) = 1 %.

EXEMPLE 35 oxydation du para,para'-diacétyldiphényléther en para,para'-dihydroxydiphényléther (double Baeyer-Villiger)

On procède comme dans l'exemple 1. Les rapports molaires sont cependant les suivants O-O /substrat = 4, meq $H^+$/substrat = 0,5.

La température est de 40° C et le substrat est ajouté en 20 minutes sous forme de solution à 20,2 % dans du dichloro-1,2-éthane.

Le temps de réaction est de 40 minutes.
TT(p,p'-MeCOØOØCOMe) = 98 %,
RT (p,p'-HOØOØOH) = 89 %.

EXEMPLE 36 oxydation de la para-hydroxy-méta-méthylacétophénone en méthylhydroquinone

On procède comme dans l'exemple 1. La température est de 25° C et le substrat est ajouté en 10 minutes sous forme d'une solution à 11,7 % pp dans du dichloro-1,2-éthane. Le temps de réaction est de 2 heures.
TT(HO-4-Me-3-ØCOMe) = 86 %,
RT(HO-4-Me-3-ØOH) = 98 %.

EXEMPLE 37 oxydation de la para-acétoxy-méta-méthylacétophénone en méthylhydroquinone

On procède comme dans l'exemple 1. La température est de 40° C et le substrat est ajouté en 10 minutes sous forme de solution à 37,7 % pp dans du dichloro-1,2-éthane. Le temps de réaction est de 2,5 heures.
TT(AcO-4-Me-3-ØCOMe) = 82 %,
RT(HO-4-Me-3-ØOH) = 88 % (les sélectivités en diacétate et en monoacétate d'hydroquinone sont respectivement de 47. et 41 %)

EXEMPLE 38 oxydation de la para-méthoxy-méta-méthylacétophénone en monométhyléther de la méthylhydroquinone

On procède comme dans l'exemple 1. La température est de 25° C et le substrat est ajouté en 6 minutes. Le temps de réaction est de 2 heures.
TT(MeO-4-Me-3-ØCOMe) = 93 %,
RT(MeO-4-Me-3ØOH) = 96 % (la sélectivité en monoacétate de monométhyléther est de 94 %).

EXEMPLE 39 oxydation de la para-méthoxy-ortho-méthylacétophénone en monométhyléther de la méthylhydroquinone

On procède comme dans l'exemple 1. La température est de 25° C et le substrat est ajouté en 6 minutes. Le temps de réaction est de 2 heures.
TT(MeO-4-Me-2ØCOMe) = 93 %,
RT méthylhydroquinone supérieur à 90 % (la sélectivité en monoacétate de monométhyléther est de 90 %).

TABLEAU 1

Essais 3 à 7

| Ex N° | Catalyseur acide | Rapports molaires | | tr (hr) | TT (%) | | RT (%) | | | b) ΣRT (%) |
|---|---|---|---|---|---|---|---|---|---|---|
| | | [O-O][a] / Subst. | Cat.$H^+$ / Subst. | | [O-O] | Substrat | Ester + Phenol | "ArOH" | $ArCO_2H$ | |
| Réf | Néant | 1,1 | 0 | 4 | 43 | 46 | 5 + 0 | 5 | 95 | 100 |
| 3 | $H_2SO_4$ | 1,2 | 0,13 | 0,8 | | 92 | 75 + 16 | 91 | 9 | 99 |
| | | | | 2,0 | 77 | 97 | 70 + 19 | 89 | 9 | 98 |
| | $H_2SO_4$ [c] | 1,1 | 0,13 | 4 | 77 | 76 | 52 + 22 | 74 | 18 | 92 |
| 4 | $MeSO_3H$ | 1,1 | 0,11 | 4 | 82 | 87 | 64 + 7 | 71 | 30 | 100 |
| 5 | $F_3CSO_3H$ | 1,2 | 0,15 | 2 | 68 | 67 | 44 + 31 | 75 | 7 | 82 |
| 6 | $F_3C\ CO_2H$ | 1,1 | 0,12 | 4 | 65 | 73 | 10 + 0 | 10 | 90 | 100 |
| 7 | $H_3PO_4$ | 1,3 | 0,15 | 4 | 65 | 84 | 11 + 2 | 13 | 86 | 99 |

a) [O-O] = mole $CH_3COO_2H$ + mole $CH_3COO_2COCH_3$
b) ΣRT = bilan sur substrat,
c) uniquement ACOH comme solvant, pas de $ClCH_2CH_2Cl$.

TABLEAU 2

Essais 8 à 16

| Ex. N° | Catalyseur acide | Rapports molaires | | tr (hr) | TT (%) | | RT (%) | | | RT (Bilan Substrat) (%) |
|---|---|---|---|---|---|---|---|---|---|---|
| | | [O-O][a) ]/ Subst. | $Cat.H^+$ / Subst. | | [O-O] | Substrat | Ester + Phenol | "ArOH" | $ArCO_2H$ | |
| Réf | Néant | 1,1 | 0 | 4 | 43 | 46 | 5 + 0 | 5 | 95 | 100 |
| 3 | $H_2SO_4$ [b)] | 1,2 | 0,13 | 2 | 77 | 97 | 70 + 19 | 89 | 9 | 98 |
| 8 | Amberlyst 15-H | 1,1 | 0,25 | 4 | 95 | 79 | 56 + 21 | 84 | 14 | 98 |
| 9 | " " | 1,1 | 0,06 | 1,8 | 79 | 71 | 76 + 4 | 80 | 16 | 96 |
| 10 | " " | 1,0 | 0,11 | 4 | 97 | 84 | 72 + 14 | 86 | 14 | 100 |
| 11 | " " | 1,1 | 0,13 [c)] | 1,8 | 87 | 74 | 70 + 9 | 79 | 20 | 99 |
| 12 | d) " " | 1,1 | 0,13 | 4 | 96 | 82 | 1 + 74 | 75 | 15 | 90 |
| 13 | e) " " | 1,2 | 0,13 | 2 | 87 | 99 | 75 + 11 | 87 | 13 | 100 |
| 14 | f) " " | 1,3 | 0,15 | 2 | 95 | 91 | 77 + 10 | 87 | 11 | 98 |
| 15 | Nafion 117-H | 1,0 | 0,12 | 4 | 86 | 58 | 29 + 43 | 72 | 11 | 83 |
| 16 | " " | 1,3 | 0,06 | 2 | 83 | 99 | 76 + 15 | 91 | 8 | 99 |

a) [O-O] = mole $CH_3COO_2H$ + mole $CH_3COO_2COCH_3$ ;
b) essais de références ;
c) catalyseur recyclé de l'exemple 10 ;
d) solvant = $CH_2Cl_2$, est éliminé après coulée du substrat ;
e) temps de coulée du substrat = 10 minutes ;
f) temps de coulée du substrat = 45 minutes.

TABLEAU 3

Essais 17 à 22

| Ex. N° | Catalyseur acide | Rapports molaires | | tr (hr) | TT (%) | | RT (%) | | | RT (Bilan Substrat) (%) |
|---|---|---|---|---|---|---|---|---|---|---|
| | | [O-O][a) / Subst. | Cat.$H^+$ / Subst. | | [O-O] | Substrat | Ester + Phenol | "ArOH" | $ArCO_2H$ | |
| 18 | Néant | 1,1 | 0 | 4,5 | 3 | 4 | 41 + 0 | 41 | 60 | 100 |
| 19 [b)] | $H_2SO_4$ | 1,1 | 0,1 | 4 | 86 | 68 | 35 + 29 | 64 | 15 | 79 |
| 20 | $MeSO_3H$ | 1,0 | 0,14 | 4 | 57 | 33 | 55 + 17 | 71 | 19 | 91 |
| 17 | $F_3CSO_3H$ | 1,5 | 0,15 | 4 | 71 | 85 | 66 + 26 | 92 | 3 | 96 |
| 21 | $F_3CCO_2H$ | 1,1 | 0,12 | 4 | 10 | 7 | 75 + 0 | 75 | - | 75 |
| 22 | $H_3PO_4$ | 1,2 | 0,17 | 4 | 5 | 8 | 87 + e | 87 | 12 | 99 |

a) [O-O] = mole $AcO_2H$ + mole $AcO_2Ac$ ;
b) uniquement AcOH comme solvant.

TABLEAU 4

Essais 23 à 30

| Ex. N° | | Catalyseur acide | Rapports molaires | | tr (hr) | TT (%) | | RT (%) | | | RT (Bilan Substrat) (%) |
|---|---|---|---|---|---|---|---|---|---|---|---|
| | | | [O-O] Subst. | Cat.H+ / Subst. | | [O-O] | Substrat | Ester + Phenol | "ArOH" | $ArCO_2H$ | |
| Réf | | Néant [a)] | 1,1 | 0 | 4,5 | 3 | 4 | 41 + 10 | 41 | 60 | 100 |
| | 19 [b)] | $H_2SO_4$ [a)] | 1,1 | 0,1 | 4 | 86 | 68 | 35 + 29 | 64 | 15 | 79 |
| | 23 | Amberlyst 15-H | 1,0 | 0,12 | 4 | 60 | 43 | 85 + 8 | 93 | 4 | 97 |
| | 24 | " " | 1,1 | 0,24 | 4 | 78 | 25,6 | 56 + 13 | 69 | 9 | 78 |
| | 17 | $F_3CSO_3H$ [a)] | 1,5 | 0,15 | 4 | 71 | 85 | 66 + 26 | 92 | 3 | 95 |
| | 25 | Nafion 117 H | 1,1 | 0,06 | 4 | 68 | 70 | 82 + 14 | 96 | 3 | 99 |
| | 26 | " " | 1,1 | 0,13 | 4 | 84 | 76 | 63 + 20 | 83 | 2 | 85 |
| | 27 | " " | 2,1 | 0,04[c] | 5 | 42 | 76 | 77 + 18 | 96 | 3 | 99 |
| | 28 | " " | 1,5 | 0,06[c] | 4 | 63 | 85 | 80 + 17 | 97 | 2 | 99 |
| | 29 | " " | 1,1 | 0,13[c] | 4 | 78 | 74 | 74 + 23 | 97 | 2 | 99 |
| | 30 | " " | 2,1 | 0,14[c] | 5 | 64 | 99 | 67 + 28 | 95 | 3 | 98 |

a) essai de référence ;
b) uniquement AcOH comme solvant ;
c) catalyseur recyclé de l'exemple 26.

## Revendications

1 - Procédé de préparation de dérivés aromatiques hydroxylés par la réaction de Baeyer-Villiger caractérisé en ce que :. dans une première étape on prépare une solution d'acide percarboxylique par addition d'anhydride carboxylique à une solution de péroxyde d'hydrogène dans le un solvant halogéné en présence d'un catalyseur acide fort,

. dans une deuxième étape on ajoute une arylcétone ou un aldéhyde aromatique à la solution précédente et on maintient le milieu à une température inférieure ou égale à 40° C.

2 - Procédé selon la revendication 1 caractérisé en ce qu'on utilise une quantité maximale de péroxyde d'hydrogène en solution aqueuse à 70 % correspondant à la formation d'une solution contenant au maximum 35 % en poids d'acide peracétique et ne contenant plus de péroxyde d'hydrogène libre.

3 - Procédé selon la revendication 2 caractérisé en ce que la quantité maximale de péroxyde d'hydrogène utilisé correspond à la formation d'une solution contenant entre 15 et 25 % en poids d'acide péracétique et de préférence contenant environ 20 %.

4 - Procédé selon la revendication 1 caractérisé en ce que les arylcétones ou les aldéhydes aromatiques sont choisis parmi les composés de formule générale (I) :

$(R')_n$-Ar-CO-R (I)

dans laquelle

Ar représente un radical mono ou polycyclique ou hétérocyclique,

R' représente un motif choisi parmi les radicaux alkyle contenant 1 à 4 atomes de carbone, alcoxy contenant 1 à 4 atomes de carbone, hydroxy, halogéno, perhalogénoalkyle, acyloxy, alcoxycarbonyl, perhalogénoalcoxy, nitro, phényl et phénoxy, éventuellement substitués par un groupe hydroxyle, halogéno, formyl, acyloxy.

n est un nombre entier égal à 0, 1 ou 2,

R est choisi parmi l'hydrogène, les groupes alkyles contenant 1 à 4 atomes de carbone, un groupe phényl.

5 - Procédé selon la revendication 4 caractérisé en ce que le radical Ar est un cycle benzénique.

6 - Procédé selon les revendications 4 et 5 caractérisé en ce que le composé de formule (I) est la fluoroacétophénone.

7. Procédé selon la revendication 1 caractérisé en ce que dans la deuxième étape on ajoute l'arylcétone en quantité telle que le rapport molaire de l'acide peracétique à l'arylcétone est compris entre 1 et 5 et de préférence entre 1,2 et 1,3.

8 - Procédé selon la revendication 1 caractérisé en ce que l'acide percarboxylique est non halogéné et contient 1 à 3 atomes de carbone.

9 - Procédé selon la revendication 8 caractérisé en ce que l'acide percarboxylique est choisi parmi l'acide performique et l'acide peracétique.

10 - Procédé selon la revendication 1 caractérisé en ce que au cours de la deuxième étape on ajoute un catalyseur acide choisi parmi l'acide trifluoroacétique, l'acide sulfurique, l'acide trifluorométhanesulfonique, l'acide méthanesulfonique, l'Amberlyst® ou le Nafion®, l'acide phosphorique.

11 - Procédé selon la revendication 8 caractérisé en ce que l'acide trifluoroacétique est choisi parmi l'acide sulfurique, l'acide trifluorométhanesulfonique et le Nafion®.

12 - Procédé selon la revendication 1 caractérisé en ce que le rapport pondéral de l'acide fort selon les revendication 8 ou 9 à l'acide percarboxylique est compris entre 5 et 20 %.